# EUROPEAN PATENT SPECIFICATION

(11) **EP 1 660 460 B1**
(45) Date of publication and mention of the grant of the patent: **26.03.2008**
(21) Application number: 04775331.4
(22) Date of filing: 25.08.2004
(51) Int. Cl.: C07D 241/44, C07D 403/12, C07D 413/12, A61K 31/498, A61P 29/00

(54) **QUINOXALINE DERIVATIVES AS NEUTROPHIL ELASTASE INHIBITORS AND THEIR USE**
CHINOXALINDERIVATE ALS INHIBITOREN DER NEUTROPHILELASTASE UND DEREN VERWENDUNG
DERIVES DE QUINOXALINE UTILISES COMME INHIBITEURS DE L'ELASTASE NEUTROPHILE ET LEUR UTILISATION

(30) Priority: 28.08.2003 SE 0302324
(43) Date of publication of application: 31.05.2006
(73) Proprietor: AstraZeneca AB, 151 85 Södertälje (SE)
(72) Inventor: BLADH, Håkan, S-221 87 Lund (SE); LARSSON, Joakim, S-221 87 Lund (SE)
(86) International application number: PCT/SE2004/001225
(87) International publication number: WO 2005/021512

(56) References cited:
- EP-A1- 0 008 864
- DATABASE CAPLUS 1995: 456529, 1994 XP002903883 & UKRAINETS I.V. ET AL.: '4-Hydroxy-2quinolones. 23.N-(2-thiazolyl) amides of 1-substituted 4-hydroxy-2-oxoquinoline-3-carboxylic acids - a new group of potential antiinflammatory drugs' 1994, pages 1397 - 9
- DATABASE CAPLUS 1990:611864 XP002903884 & JP 02 152 966 A

## Description

### Field of the Invention

This invention relates to novel quinoxaline derivatives, processes for their preparation, pharmaceutical compositions comprising them, and their use in therapy.

### Background of the Invention

Elastases are possibly the most destructive enzymes in the body, having the ability to degrade virtually all connective tissue components. The uncontrolled proteolytic degradation by elastases has been implicated in a number of pathological conditions. Human neutrophil elastase (hNE), a member of the chymotrypsin superfamily of serine proteases is a 33-KDa enzyme stored in the azurophilic granules of the neutrophils. In neutrophils the concentration of NE exceeded 5 mM and its total cellular amount has been estimated to be up to 3 pg. Upon activation, NE is rapidly released from the granules into the extracellular space with some portion remaining bound to neutrophil plasma membrane (See Kawabat et al. 2002, Eur. J. Pharmacol. 451, 1-10). The main intracellular physiological function of NE is degradation of foreign organic molecules phagocytosed by neutrophils, whereas the main target for extracellular elastase is elastin (Janoff and Scherer, 1968, J. Exp. Med. 128, 1137-1155). NE is unique, as compared to other proteases (for example, proteinase 3) in that it has the ability to degrade almost all extracellular matrix and key plasma proteins (See Kawabat et al., 2002, Eur. J. Pharmacol. 451, 1-10). It degrades a wide range of extracellular matrix proteins such as elastin, Type 3 and type 4 collagens, laminin, fibronectin, cytokines, etc. (Ohbayashi, H., 2002, Expert Opin. Investig. Drugs, 11, 965-980). NE is a major common mediator of many pathological changes seen in chronic lung disease including epithelial damage (Stockley, R.A. 1994, Am. J. Resp. Crit. Care Med. 150, 109-113).

The destructive role of NE was solidified almost 40 years ago when Laurell and Eriksson reported an association of chronic airflow obstruction and emphysema with deficiency of serum α₁-antitrypsin (Laurell and Eriksson, 1963, Scand. J. Clin. Invest. 15, 132-140). Subsequently it was determined that α₁-antitrypsin is the most important endogenous inhibitor of human NE. The imbalance between human NE and endogenous antiprotease is believed to cause excess human NE in pulmonary tissues which is considered as a major pathogenic factor in chronic obstructive pulmonary disease (COPD). The excessive human NE shows a prominent destructive profile and actively takes part in destroying the normal pulmonary structures, followed by the irreversible enlargement of the respiratory airspaces, as seen mainly in emphysema. There is an increase in neutrophil recruitment into the lungs which is associated with increased lung elastase burden and emphysema in α₁-proteinase inhibitor-deficient mice (Cavarra et al., 1996, Lab. Invest. 75, 273-280). Individuals with higher levels of the NE-α₁ protease inhibitor complex in bronchoalveolar lavage fluid show significantly accelerated decline in lung functions compared to those with lower levels (Betsuyaku et al. 2000, Respiration, 67, 261-267). Instillation of human NE via the trachea in rats causes lung haemorrhage, neutrophil accumulation during acute phase and emphysematous changes during chronic phase (Karaki et al., 2002, Am. J. Resp. Crit. Care Med., 166, 496-500). Studies have shown that the acute phase of pulmonary emphysema and pulmonary haemorrhage caused by NE in hamsters can be inhibited by pre-treatment with inhibitors of NE (Fujie et al.,1999, Inflamm. Res. 48, 160-167).

Neutrophil-predominant airway inflammation and mucus obstruction of the airways are major pathologic features of COPD, including cystic fibrosis and chronic bronchitis. NE impairs mucin production, leading to mucus obstruction of the airways. NE is reported to increase the expression of major respiratory mucin gene, MUC5AC (Fischer, B.M & Voynow, 2002, Am. J. Respir. Cell Biol., 26, 447-452). Aerosol administration of NE to guinea pigs produces extensive epithelial damage within 20 minutes of contact (Suzuki et al., 1996, Am. J. Resp. Crit. Care Med., 153, 1405-1411). Furthermore NE reduces the ciliary beat frequency of human respiratory epithelium *in vitro* (Smallman et al., 1984, Thorax, 39, 663-667) which is consistent with the reduced mucociliary clearance that is seen in COPD patients (Currie et al., 1984, Thorax, 42, 126-130). The instillation of NE into the airways leads to mucus gland hyperplasia in hamsters (Lucey et al., 1985, Am. Resp. Crit. Care Med., 132, 362-366). A role for NE is also implicated in mucus hypersecretion in asthma. In an allergen sensitised guinea pig acute asthma model an inhibitor of NE prevented goblet cell degranulation and mucus hypersecretion (Nadel et al., 1999, Eur. Resp. J., 13, 190-196).

NE has been also shown to play a role in the pathogenesis of pulmonary fibrosis. NE: α₁-protenase inhibitor complex is increased in serum of patients with pulmonary fibrosis, which correlates with the clinical parameters in these patients (Yamanouchi et al., 1998, Eur. Resp. J. 11, 120-125). In a murine model of human pulmonary fibrosis, a NE inhibitor reduced bleomycin-induced pulmonary fibrosis (Taooka et al., 1997, Am. J. Resp. Crit. Care Med., 156, 260-265). Furthermore investigators have shown that NE deficient mice are resistant to bleomycin-induced pulmonary fibrosis (Dunsmore et al., 2001, Chest, 120, 35S-36S). Plasma NE level was found to be elevated in patients who progressed to ARDS implicating the importance of NE in early ARDS disease pathogenesis. (Donnelly et al., 1995, Am. J. Res. Crit. Care Med., 151, 428-1433). The antiproteases and NE complexed with antiprotease are increased in lung cancer area (Marchandise et al., 1989, Eur. Resp. J. 2, 623-629). Recent studies have shown that polymorphism in the promoter region of the NE gene are associated with lung cancer development (Taniguchi et al., 2002, Clin. Cancer Res., 8, 1115-1120.

Acute lung injury caused by endotoxin in experimental animals is associated with elevated levels of NE (Kawabata, et al., 1999, Am. J. Resp. Crit. Care, 161, 2013-2018). Acute lung inflammation caused by intratracheal injection of lipopolysaccharide in mice has been shown to elevate the NE activity in bronchoalveolar lavage fluid which is significantly inhibited by a NE inhibitor (Fujie et al., 1999, Eur. J. Pharmacol., 374, 117-125; Yasui, et al., 1995, Eur. Resp. J., 8, 1293-1299). NE also plays an important role in the neutrophil-induced increase of pulmonary microvascular permeability observed in a model of acute lung injury caused by tumour necrosis factor α (TNFα) and phorbol myristate acetate (PMA) in isolated perfused rabbit lungs (Miyazaki et al., 1998, Am. J. Respir. Crit. Care Med., 157, 89-94).

A role for NE has also been suggested in monocrotoline-induced pulmonary vascular wall thickening and cardiac hypertrophy (Molteni et al., 1989, Biochemical Pharmacol. 38, 2411-2419). Serine elastase inhibitor reverses the monocrotaline-induced pulmonary hypertension and remodelling in rat pulmonary arteries (Cowan et al., 2000, Nature Medicine, 6, 698-702). Recent studies have shown that serine elastase, that is, NE or vascular elastase are important in cigarette smoke-induced muscularisation of small pulmonary arteries in guinea pigs (Wright et al., 2002, Am. J. Respir. Crit. Care Med., 166, 954-960).

NE plays a key role in experimental cerebral ischemic damage (Shimakura et al., 2000, Brain Research, 858, 55-60), ischemia-reperfusion lung injury (Kishima et al., 1998, Ann. Thorac. Surg. 65, 913-918) and myocardial ischemia in rat heart (Tiefenbacher et al., 1997, Eur. J. Physiol., 433, 563-570). Human NE levels in plasma are significantly increased above normal in inflammatory bowel diseases, for example, Crohn's disease and ulcerative colitis (Adeyemi et al., 1985, Gut, 26, 1306-1311). In addition NE has also been assumed to be involved in the pathogenesis of rheumatoid arthritis (Adeyemi et al., 1986, Rheumatol. Int., 6, 57). The development of collagen induced arthritis in mice is suppressed by a NE inhibitor (Kakimoto et al., 1995, Cellular Immunol. 165, 26-32).

Thus, human NE is known as one of the most destructive serine proteases and has been implicated in a variety of inflammatory diseases. The important endogenous inhibitor of human NE is α₁-antitrypsin. The imbalance between human NE and antiprotease is believed to give rise to an excess of human NE resulting in uncontrolled tissue destruction. The protease/ antiprotease balance may be upset by a decreased availability of α₁-antitrypsin either through inactivation by oxidants such as cigarette smoke, or as a result of genetic inability to produce sufficient serum levels. Human NE has been implicated in the promotion or exacerbation of a number of diseases such as pulmonary emphysema, pulmonary fibrosis, adult respiratory distress syndrome (ARDS), ischemia reperfusion injury, rheumatoid arthritis and pulmonary hypertension.

EP 0 008 864 discloses certain pyridopyrazine and quinoxaline derivatives useful as antiinflammatory agents.

The present invention discloses novel quinoxaline derivatives that are inhibitors of human neutrophil elastase and homologous serine proteases such as proteinase 3 and pancreatic elastase, and are thereby useful in therapy.

### Disclosure of the Invention

The present invention provides a compound of formula (I) 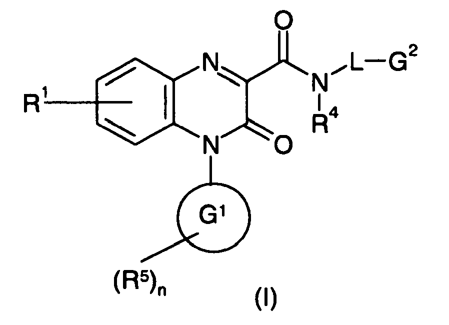 wherein
**R¹** represents H, halogen, CN, C1 to 6 alkyl, C1 to 6 alkoxy, CO₂R⁷ or CONR⁸R⁹;
**G¹** represents phenyl or a five- or six-membered heteroaromatic ring containing 1 to 3 heteroatoms independently selected from O, S and N;
**R⁵** represents H, halogen, C1 to 6 alkyl, CN, C1 to 6 alkoxy, NO₂, NR¹⁴R¹⁵, C1 to 3 alkyl substituted by one or more F atoms or C1 to 3 alkoxy substituted by one or more F atoms;
**R¹⁴** and **R¹⁵** independently represent H or C1 to 3 alkyl; said alkyl being optionally further substituted by one or more F atoms;
**n** represents an integer 1, 2 or 3 and when n represents 2 or 3, each R⁵ group is selected independently;
**R⁴** represents H or C1 to 6 alkyl; said alkyl being optionally further substituted by OH or C1 to 6 alkoxy;
or **R⁴** and **L** are joined together such that the group **-NR⁴L** represents a 5 to 7 membered azacyclic ring optionally incorporating one further heteroatom selected from O, S and NR¹⁶;
**L** represents a bond, O, NR²⁹ or C1 to 6 alkyl; said alkyl being optionally incorporating a heteroatom selected from O, S and NR¹⁶; and said alkyl being optionally further substituted by OH or OMe;
**G²** represents a monocyclic ring system selected from:
   i) phenyl or phenoxy,
   ii) a 5 or 6 membered heteroaromatic ring containing one to three heteroatoms independently selected from O, S and N,
   iii) a C3 to 6 saturated or partially unsaturated cycloalkyl, or
   iv) a C4 to 7 saturated or partially unsaturated heterocyclic ring containing one or two heteroatoms independently selected from O, S(O)ₚ and NR¹⁷ and optionally further incorporating a carbonyl group; or
**G²** represents a bicyclic ring system in which each of the two rings is independently selected from:
   i) phenyl,
   ii) a 5 or 6 membered heteroaromatic ring containing one to three heteroatoms independently selected from O, S and N,
   iii) a C3 to 6 saturated or partially unsaturated cycloalkyl, or
   iv) a C4 to 7 saturated or partially unsaturated heterocyclic ring containing one or two heteroatoms independently selected from O, S(O)ₚ and NR¹⁷ and optionally further incorporating a carbonyl group;
and the two rings are either fused together, or are bonded directly together or are separated by a linker group selected from O, S(O)_{q} or CH₂,
said monocyclic or bicyclic ring system being optionally further substituted by one to three substituents independently selected from CN, OH, C1 to 6 alkyl, C1 to 6 alkoxy, halogen, NR¹⁸R¹⁹, NO₂, OSO₂R³⁸, CO₂R²⁰, C(=NH)NH₂, C(O)NR²¹R²², C(S)NR²³R²⁴, SC(=NH)NH₂, NR³¹C(=NH)NH₂, S(O)ₛR²⁵, SO₂NR²⁶R²⁷, C1 to 3 alkoxy substituted by one or more F atoms and C1 to 3 alkyl substituted by SO₂R³⁹ or by one or more F atoms; or
when L does not represent a bond, **G²** may also represent H;
**p, q, s** and **t** independently represent an integer 0, 1 or 2;
**R⁸** and **R⁹** independently represent H or C1 to 6 alkyl; or the group **NR⁸R⁹** together represents a 5 to 7 membered azacyclic ring optionally incorporating one further heteroatom selected from O, S and NR²⁸;
**R¹⁸** and **R¹⁹** independently represent H, C1 to 6 alkyl, formyl, C2 to 6 alkanoyl, S(O)ₜR³² or SO₂NR³³R³⁴; said alkyl group being optionally further substituted by halogen, CN, C1 to 4 alkoxy or CONR⁴¹R⁴²;
**R²⁵** represents H, C1 to 6 alkyl or C3 to 6 cycloalkyl; said alkyl group being optionally further substituted by one or more substituents selected independently from OH, CN, CONR³⁵R³⁶, CO₂R³⁷, OCOR⁴⁰, C3 to 6 cycloalkyl, a C4 to 7 saturated heterocyclic ring containing one or two heteroatoms independently selected from O, S(O)ₚ and NR⁴³ and phenyl or a 5 or 6 membered heteroaromatic ring containing one to three heteroatoms independently selected from O, S and N; said aromatic ring being optionally further substituted by one or more substituents selected independently from halogen, CN, C1 to 4 alkyl, C1 to 4 alkoxy, OH, CONR⁴⁴R⁴⁵, CO2R⁴⁶, S(O)ₛR²⁵ or NHCOCH₃;
**R³²** represents H, C1 to 6 alkyl or C3 to 6 cycloalkyl;
**R⁷, R¹⁶, R¹⁷, R²⁰, R²¹, R²², R²³, R²⁴, R²⁶, R²⁷, R²⁸, R²⁹, R³¹, R³³, R³⁴, R³⁵, R³⁶, R³⁷, R³⁸, R³⁹, R⁴⁰, R⁴¹, R⁴², R⁴³, R⁴⁴, R⁴⁵ and R⁴⁶** independently represent H or C1 to 6 alkyl;
and pharmaceutically acceptable salts thereof.

The compounds of formula (I) may exist in enantiomeric and/or tautomeric forms. It is to be understood that all enantiomers, diastereomers, racemates, tautomers and mixtures thereof are included within the scope of the invention.

Unless otherwise indicated, the term "C1 to 6 alkyl" referred to herein denotes a straight or branched chain alkyl group having from 1 to 6 carbon atoms. Examples of such groups include methyl, ethyl, n-propyl, i-propyl, n-butyl, i-butyl, t-butyl, pentyl and hexyl. The terms "C1 to 3 alkyl" and "C1 to 4 alkyl" are to be interpreted analogously.

Examples of "C1 to 3 alkyl substituted by one or more F atoms" include fluoromethyl, difluoromethyl, trifluoromethyl, 2,2,2-trifluoroethyl, 1,1-difluoroethyl, pentafluoroethyl and 3,3,3-trifluoropropyl.

Unless otherwise indicated, the term "C1 to 6 alkoxy " referred to herein denotes an oxygen substituent bonded to a straight or branched chain alkyl group having from 1 to 6 carbon atoms. Examples of such groups include methoxy, ethoxy, n-propoxy, i-propoxy, n-butoxy, i-butoxy and s-butoxy. The terms "C1 to 3 alkoxy" and "C1 to 4 alkoxy" are to be interpreted analogously.

Examples of "C1 to 3 alkoxy substituted by one or more F atoms" include fluoromethoxy, trifluoromethoxy, 2,2,2-trifluoroethoxy and 3,3,3-trifluoropropoxy.

Unless otherwise indicated, the term "C2 to 6 alkanoyl" referred to herein denotes a straight or branched chain alkyl group having from 1 to 5 carbon atoms bonded to the molecule via a carbonyl group. Examples of such groups include acetyl, propionyl and pivaloyl.

Unless otherwise indicated, the term "halogen" referred to herein denotes fluorine, chlorine, bromine and iodine.

Examples of a five or six membered heteroaromatic ring containing 1 to 3 heteroatoms independently selected from O, S and N include furan, thiophene, pyrrole, oxazole, oxadiazole, isoxazole, imidazole, thiazole, triazole, thiadiazole, pyridine, pyrimidine and pyrazine.

Unless otherwise indicated, the term "C3 to 6 saturated or partially unsaturated cycloalkyl" referred to herein denotes a 3 to 6 membered non-aromatic carbocyclic ring optionally incorporating one or more double bonds. Examples include cyclopropyl, cyclopentyl, cyclopentenyl, cyclohexyl and cyclohexenyl. The term "five- or six-membered saturated or partially unsaturated cycloalkyl ring" is to be interpreted analogously.

Unless otherwise indicated, the term "C4 to 7 saturated or partially unsaturated heterocyclic ring containing one or two heteroatoms independently selected from O, S(O)ₚ and NR¹⁷ and optionally further incorporating a carbonyl group" referred to herein denotes a 4 to 7 membered non-aromatic heterocyclic ring optionally incorporating one or more double bonds and optionally incorporating a carbonyl group. Examples include tetrahydrofuran, thiolane 1,1-dioxide, tetrahydropyran, 4-oxo-4H-pyran, pyrrolidine, pyrroline, imidazolidine, 1,3-dioxolane, piperidine, piperazine, morpholine, perhydroazepine, pyrrolidone and piperidone. The term "five- or six-membered saturated or partially unsaturated heterocyclic ring containing one heteroatom selected from O, S and NR¹³" is to be interpreted analogously.

Examples of a "5 to 7 membered azacyclic ring optionally incorporating one further heteroatom selected from O, S and NR¹⁶" include pyrrolidine, piperidine, morpholine, thiomorpholine and piperazine.

In the definition of L, "C1 to 6 alkyl; said alkyl optionally incorporating a heteroatom selected from O, S and NR¹⁶" embraces a straight or branched chain arrangement of 1 to 6 carbon atoms in which any two carbon atoms are optionally separated by O, S or NR¹⁶. The definition thus includes, for example, methylene, ethylene, propylene, hexamethylene, ethylethylene, -CH₂CH₂O-CH₂-, -CH₂CH₂O-CH₂-CH₂-, -CH₂CH₂S- and -CH₂CH₂NR¹⁶-.

Examples of bicyclic ring systems in which the two rings are either fused together, or are bonded directly together or are separated by a linker group selected from O, S(O)_{q} or CH₂ include biphenyl, thienylphenyl, pyrazolylphenyl, phenoxyphenyl, naphthyl, indanyl, quinolyl, tetrahydroquinolyl, benzofuranyl, indolyl, isoindolyl, indolinyl, benzofuranyl, benzothienyl, indazolyl, benzimidazolyl, benzthiazolyl, purinyl, isoquinolyl, chromanyl, indenyl, quinazolyl, quinoxalyl, chromanyl, isocromanyl, 3H-indolyl, 1H-indazolyl, quinuclidyl, tetrahydronaphthyl, dihydrobenzofuranyl, morpholine-4-ylphenyl, 1,3-benzodioxolyl, 1,1-dioxido-2,3-dihydro-1-benzothienyl, 2,3-dihydro-1,4-benzodioxinyl and 3,4-dihydro-isochromenyl.

In one embodiment, R¹ in formula (I) represents H.

In one embodiment, G¹ in formula (I) represents phenyl or pyridyl. In another embodiment, G¹ in formula (I) represents phenyl.

In one embodiment, R⁵ in formula (I) represents halogen, C1 to 6 alkyl, CN or C 1 to 3 alkyl substituted by one or more F atoms. In another embodiment, R⁵ in formula (I) represents Cl, CH₃, CN or CF₃.

In one embodiment, n represents the integer 1.

In another embodiment, G¹ in formula (I) represents phenyl, R⁵ represents CF₃ and n represents the integer 1.

In one embodiment, R⁴ represents H.

In one embodiment, L represents C1 to 6 alkyl. In another embodiment, L represents -CH₂-. In another embodiment, L represents NR²⁹ and R²⁹ represents H.

In one embodiment, G² represents an optionally substituted monocyclic ring system selected from:
i) phenyl,
ii) a 5 or 6 membered heteroaromatic ring containing one to three heteroatoms independently selected from O, S and N,
iii) a C3 to 6 saturated or partially unsaturated cycloalkyl, or
iv) a C4 to 7 saturated or partially unsaturated heterocyclic ring containing one or two heteroatoms independently selected from O, S(O)ₚ and NR¹⁷ and optionally further incorporating a carbonyl group.

In another embodiment, G² represents optionally substituted phenyl. In another embodiment, G² represents phenyl substituted by OSO₂R³⁸, S(O)ₛR²⁵, SO₂NR²⁶R²⁷, NR¹⁸R¹⁹ (wherein at least one of R¹⁸ and R¹⁹ represents S(O)ₜR³² or SO2NR³³R³⁴) or C1 to 3 alkyl substituted by SO₂R³⁹.

In another embodiment, G² represents an optionally substituted bicyclic ring system in which each of the two rings is independently selected from:
i) phenyl,
ii) a 5 or 6 membered heteroaromatic ring containing one to three heteroatoms independently selected from O, S and N,
iii) a C3 to 6 saturated or partially unsaturated cycloalkyl, or
iv) a C4 to 7 saturated or partially unsaturated heterocyclic ring containing one or two heteroatoms independently selected from O, S(O)ₚ and NR¹⁷ and optionally further incorporating a carbonyl group;
and the two rings are either fused together, or are bonded directly together or are separated by a linker group selected from O, S(O)_{q} or CH₂.

In one embodiment, R¹ in formula (I) represents H; G¹ represents phenyl; R⁵ represents halogen, C1 to 6 alkyl, CN or C1 to 3 alkyl substituted by one or more F atoms; R⁴ represents H; L represents C1 to 6 alkyl; and G² represents an optionally substituted monocyclic ring system selected from:
i) phenyl,
ii) a 5 or 6 membered heteroaromatic ring containing one to three heteroatoms independently selected from O, S and N,
iii) a C3 to 6 saturated or partially unsaturated cycloalkyl, or
iv) a C4 to 7 saturated or partially unsaturated heterocyclic ring containing one or two heteroatoms independently selected from O, S(O)ₚ and NR¹⁷ and optionally further incorporating a carbonyl group.

In another aspect, the invention specifically provides any compound as described in the Examples herein, or the free base thereof or a pharmaceutically acceptable salt thereof. Particular compounds include:
N-(4-chlorobenzyl)-3-oxo-4-[3-(trifluoromethyl)phenyl]-3,4-dihydroquinoxaline-2-carboxamide;
4-(3-cyanophenyl)-*N*-[4-(methylsulfonyl)benzyl]-3-oxo-3,4-dihydroquinoxaline-2-carboxamide;
4-(3-chlorophenyl)-*N*-[4-(methylsulfonyl)benzyl]-3-oxo-3,4-dihydroquinoxaline-2-carboxamide;
4-(3-methylphenyl)-*N*-[4-(methylsulfonyl)benzyl]-3-oxo-3,4-dihydroquinoxaline-2-carboxamide;
N-[4-(methylsulfonyl)benzyl]-3-oxo-4-[3-(trifluoromethyl)phenyl]-3,4-dihydroquinoxaline-2-carboxamide;
N-(cyclohexylmethyl)-3-oxo-4-[3-(trifluoromethyl)phenyl]-3,4-dihydroquinoxaline-2-carboxamide;
N-[2-(3,4-dimethoxyphenyl)ethyl]-3-oxo-4-[3-(trifluoromethyl)phenyl]-3,4-dihydroquinoxaline-2-carboxamide;
3-oxo-N-(pyridin-3-ylmethyl)-4-[3-(trifluoromethyl)phenyl]-3,4-dihydroquinoxaline-2-carboxamide;
3-oxo-N'-phenyl-4-[3-(trifluoromethyl)phenyl]-3,4-dihydroquinoxaline-2-carbohydrazide; N-(3-bromobenzyl)-3-oxo-4-[3-(trifluoromethyl)phenyl]-3,4-dihydroquinoxaline-2-carboxamide;
N-(4-bromobenzyl)-3-oxo-4-[3-(trifluoromethyl)phenyl]-3,4-dihydroquinoxaline-2-carboxamide;
3-oxo-N-[(2R)-2-phenylcyclopropyl]-4-[3-(trifluoromethyl)phenyl]-3,4-dihydroquinoxaline-2-carboxamide;
N-[2-(3-chlorophenyl)ethyl]-3-oxo-4-[3-(trifluoromethyl)phenyl]-3,4-dihydroquinoxaline-2-carboxamide;
N-[(4-cyanocyclohexyl)methyl]-3-oxo-4-[3-(trifluoromethyl)phenyl]-3,4-dihydroquinoxaline-2-carboxamide;
N-(1-naphthylmethyl)-3-oxo-4-[3-(trifluoromethyl)phenyl]-3,4-dihydroquinoxaline-2-carboxamide;
N-(4-methylbenzyl)-3-oxo-4-[3-(trifluoromethyl)phenyl]-3,4-dihydroquinoxaline-2-carboxamide;
N-(1,3-benzodioxol-5-ylmethyl)-3-oxo-4-[3-(trifluoromethyl)phenyl]-3,4-dihydroquinoxaline-2-carboxamide;
N-(2,4-dichlorobenzyl)-3-oxo-4-[3-(trifluoromethyl)phenyl]-3,4-dihydroquinoxaline-2-carboxamide;
N-(4-methoxybenzyl)-3-oxo-4-[3-(trifluoromethyl)phenyl]-3,4-dihydroquinoxaline-2-carboxamide;
N-(3,4-difluorobenzyl)-3-oxo-4-[3-(trifluoromethyl)phenyl]-3,4-dihydroquinoxaline-2-carboxamide;
N-(2-chloro-4-fluorobenzyl)-3-oxo-4-[3-(trifluoromethyl)phenyl]-3,4-dihydroquinoxaline-2-carboxamide;
N-(3,4-dichlorobenzyl)-3-oxo-4-[3-(trifluoromethyl)phenyl]-3,4-dihydroquinoxaline-2-carboxamide;
N-[2-(3-methoxyphenyl)ethyl]-3-oxo-4-[3-(trifluoromethyl)phenyl]-3,4-dihydroquinoxaline-2-carboxamide;
N-[2-(4-fluorophenyl)ethyl]-3-oxo-4-[3-(trifluoromethyl)phenyl]-3,4-dihydroquinoxaline-2-carboxamide;
N-[1-(4-chlorophenyl)ethyl]-3-oxo-4-[3-(trifluoromethyl)phenyl]-3,4-dihydroquinoxaline-2-carboxamide;
3-oxo-N-[3-(2-oxopyrrolidin-1-yl)propyl]-4-[3-(trifluoromethyl)phenyl]-3,4-dihydroquinoxaline-2-carboxamide;
3-oxo-N-(pyridin-4-ylmethyl)-4-[3-(trifluoromethyl)phenyl]-3,4-dihydroquinoxaline-2-carboxamide;
N-(2,3-dihydro-1-benzofuran-5-ylmethyl)-3-oxo-4-[3-(trifluoromethyl)phenyl]-3,4-dihydroquinoxaline-2-carboxamide;
methyl 4-{[({3-oxo-4-[3-(trifluoromethyl)phenyl]-3,4-dihydroquinoxalin-2-yl}carbonyl)amino]methyl}benzoate;
3-oxo-N-(4-phenoxybenzyl)-4-[3-(trifluoromethyl)phenyl]-3,4-dihydroquinoxaline-2-carboxamide;
N-{2-[4-(aminosulfonyl)phenyl]ethyl}-3-oxo-4-[3-(trifluoromethyl)phenyl]-3,4-dihydroquinoxaline-2-carboxamide;
3-oxo-N-[4-(1H-pyrazol-1-yl)benzyl]-4-[3-(trifluoromethyl)phenyl]-3,4-dihydroquinoxaline-2-carboxamide;
3-oxo-N-phenoxy-4-[3-(trifluoromethyl)phenyl]-3,4-dihydroquinoxaline-2-carboxamide; N-{[3-(4-methoxyphenyl)isoxazol-5-yl]methyl}-3-oxo-4-[3-(trifluoromethyl)phenyl]-3,4-dihydroquinoxaline-2-carboxamide;
N-(3-azepan-1-ylpropyl)-3-oxo-4-[3-(trifluoromethyl)phenyl]-3,4-dihydroquinoxaline-2-carboxamide;
N-[4-(acetylamino)benzyl]-3-oxo-4-[3-(trifluoromethyl)phenyl]-3,4-dihydroquinoxaline-2-carboxamide;
N-(4-cyanobenzyl)-3-oxo-4-[3-(trifluoromethyl)phenyl]-3,4-dihydroquinoxaline-2-carboxamide;
N'-[4-(methylsulfonyl)phenyl]-3-oxo-4-[3-(trifluoromethyl)phenyl]-3,4-dihydroquinoxaline-2-carbohydrazide;
N-[4-(methylsulfonyl)phenoxy]-3-oxo-4-[3-(trifluoromethyl)phenyl]-3,4-dihydroquinoxaline-2-carboxamide;
and pharmaceutically acceptable salts thereof.

The present invention includes compounds of formula (I) in the form of salts, in particular acid addition salts. Suitable salts include those formed with both organic and inorganic acids. Such acid addition salts will normally be pharmaceutically acceptable although salts of non-pharmaceutically acceptable acids may be of utility in the preparation and purification of the compound in question. Thus, preferred salts include those formed from hydrochloric, hydrobromic, sulphuric, phosphoric, citric, tartaric, lactic, pyruvic, acetic, succinic, fumaric, maleic, methanesulphonic and benzenesulphonic acids.

In a further aspect the invention provides a process for the preparation of a compound of formula (I) which comprises reacting a compound of formula (II) 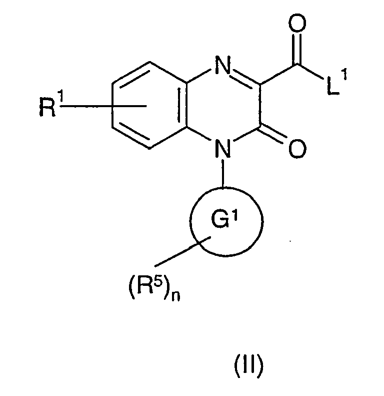 wherein R¹, R⁵, G¹ and n are as defined in formula (I) and L¹ represents a leaving group, with an amine of formula (III) or a salt thereof 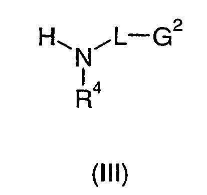 wherein R⁴, G² and L are as defined in formula (I),
and where desired or necessary converting the resultant compound of formula (I), or another salt thereof, into a pharmaceutically acceptable salt thereof; or converting one compound of formula (I) into another compound of formula (I); and where desired converting the resultant compound of formula (I) into an optical isomer thereof.

The process is carried out at a suitable temperature, generally between 0 °C and the boiling point of the solvent, in a suitable solvent such as dichloromethane or NMP (N-methylpyrrolidinone). The process is optionally carried out in the presence of a base and/or a coupling reagent such as HATU, HOAT, HOBT or DIEA. Suitable leaving groups L¹ include OH and halogen, particularly OH.

Compounds of formula (II) wherein L¹ represents OH may be prepared using methods that will be readily apparent to the man skilled in the art. See, for example, Hyrayama Takashi et al., J. Chem. Soc. Perkin Trans. 1, 1987, 75-84.

Salts of compounds of formula (I) may be formed by reacting the free base or a salt, enantiomer, tautomer or protected derivative thereof, with one or more equivalents of the appropriate acid. The reaction may be carried out in a solvent or medium in which the salt is insoluble, or in a solvent in which the salt is soluble followed by subsequent removal of the solvent *in vacuo* or by freeze drying. Suitable solvents include, for example, water, dioxane, ethanol, 2-propanol, tetrahydrofuran or diethyl ether, or mixtures thereof. The reaction may be a metathetical process or it may be carried out on an ion exchange resin.

Compounds of formula (I) and intermediate compounds thereto may be prepared as such or in protected form. The protection and deprotection of functional groups is, for example, described in 'Protective Groups in Organic Chemistry', edited by J. W. F. McOmie, Plenum Press (1973), and 'Protective Groups in Organic Synthesis', 3rd edition, T. W. Greene & P. G. M. Wuts, Wiley-Interscience (1999).

The compounds of the invention and intermediates may be isolated from their reaction mixtures, and if necessary further purified, by using standard techniques.

The compounds of formula (I) may exist in enantiomeric or diastereoisomeric forms or mixtures thereof, all of which are included within the scope of the invention. The various optical isomers may be isolated by separation of a racemic mixture of the compounds using conventional techniques, for example, fractional crystallisation or HPLC. Alternatively, the individual enantiomers may be made by reaction of the appropriate optically active starting materials under reaction conditions that will not cause racemisation.

Intermediate compounds may also exist in enantiomeric forms and may be used as purified enantiomers, diastereomers, racemates or mixtures thereof.

According to a further aspect of the invention we provide a compound of formula (I) or a pharmaceutically acceptable salt thereof, for use as a medicament.

The compounds of formula (I), and their pharmaceutically acceptable salts, are useful because they possess pharmacological activity in animals. The compounds of formula (I) have activity as pharmaceuticals, in particular as modulators of human neutrophil elastase and homologous serine proteases such as proteinase 3 and pancreatic elastase, and as such are predicted to be useful in therapy. The compounds of formula (I) are particularly useful as inhibitors of human neutrophil elastase. They may thus be used in the treatment or prophylaxis of inflammatory diseases and conditions.

Examples of these conditions are: adult respiratory distress syndrome (ARDS), cystic fibrosis, pulmonary emphysema, chronic obstructive pulmonary disease (COPD) and ischaemic-reperfusion injury. The compounds of this invention may also be useful in the modulation of endogenous and/or exogenous biological irritants which cause and/or propagate atherosclerosis, diabetes, myocardial infarction; hepatic disorders including but not limited to cirrhosis, systemic lupus erythematous, inflammatory disease of lymphoid origin, including but not limited to T lymphocytes, B lymphocytes, thymocytes; autoimmune diseases, bone marrow; inflammation of the joint (especially rheumatoid arthritis, osteoarthritis and gout); inflammation of the gastro-intestinal tract (especially inflammatory bowel disease, ulcerative colitis, pancreatitis and gastritis); inflammation of the skin (especially psoriasis, eczema, dermatitis); in tumour metastasis or invasion; in disease associated with uncontrolled degradation of the extracellular matrix such as osteoarthritis; in bone resorptive disease (such as osteoporosis and Paget's disease); diseases associated with aberrant angiogenesis; the enhanced collagen remodelling associated with diabetes, periodontal disease (such as gingivitis), corneal ulceration, ulceration of the skin, post-operative conditions (such as colonic anastomosis) and dermal wound healing; demyelinating diseases of the central and peripheral nervous systems (such as multiple sclerosis); age related illness such as dementia, inflammatory diseases of cardiovascular origins; granulomatous diseases; renal diseases including but not limited to nephritis and polyarteritis; cancer; pulmonary hypertension, ingested poisons, skin contacts, stings, bites; asthma; rhinitis; HIV disease progression; for minimising the effects of organ rejection in organ transplantation including but not limited to human organs; and replacement therapy of proteinase inhibitors.

In another aspect, the invention provides the use of a compound of formula (I) or a pharmaceutically acceptable salt thereof, in the manufacture of a medicament for the treatment or prophylaxis of inflammatory diseases or conditions.

In particular, the compounds of this invention may be used in the treatment of adult respiratory distress syndrome (ARDS), cystic fibrosis, pulmonary emphysema, chronic obstructive pulmonary disease (COPD), pulmonary hypertension, asthma, rhinitis, ischemia-reperfusion injury, rheumatoid arthritis, osteoarthritis, cancer, atherosclerosis and gastric mucosal injury.

Prophylaxis is expected to be particularly relevant to the treatment of persons who have suffered a previous episode of, or are otherwise considered to be at increased risk of, the disease or condition in question. Persons at risk of developing a particular disease or condition generally include those having a family history of the disease or condition, or those who have been identified by genetic testing or screening to be particularly susceptible to developing the disease or condition.

For the above mentioned therapeutic indications, the dose of the compound to be administered will depend on the compound employed, the disease being treated, the mode of administration, the age, weight and sex of the patient. Such factors may be determined by the attending physician. However, in general, satisfactory results are obtained when the compounds are administered to a human at a daily dosage of between 0.1 mg/kg to 100 mg/kg (measured as the active ingredient).

The compounds of formula (I) may be used on their own, or in the form of appropriate pharmaceutical formulations comprising the compound of the invention in combination with a pharmaceutically acceptable diluent, adjuvant or carrier. Particularly preferred are compositions not containing material capable of causing an adverse reaction, for example, an allergic reaction. Conventional procedures for the selection and preparation of suitable pharmaceutical formulations are described in, for example, "Pharmaceuticals - The Science of Dosage Form Designs", M. E. Aulton, Churchill Livingstone, 1988.

According to the invention, there is provided a pharmaceutical formulation comprising preferably less than 95% by weight and more preferably less than 50% by weight of a compound of formula (I) in admixture with a pharmaceutically acceptable diluent or carrier.

We also provide a method of preparation of such pharmaceutical formulations that comprises mixing the ingredients.

The compounds may be administered topically, for example, to the lungs and/or the airways, in the form of solutions, suspensions, HFA aerosols or dry powder formulations, for example, formulations in the inhaler device known as the Turbuhaler^{®}; or systemically, for example, by oral administration in the form of tablets, pills, capsules, syrups, powders or granules; or by parenteral administration, for example, in the form of sterile parenteral solutions or suspensions; or by rectal administration, for example, in the form of suppositories.

Dry powder formulations and pressurized HFA aerosols of the compounds of the invention may be administered by oral or nasal inhalation. For inhalation, the compound is desirably finely divided. The finely divided compound preferably has a mass median diameter of less than 10 µm, and may be suspended in a propellant mixture with the assistance of a dispersant, such as a C₈-C₂₀ fatty acid or salt thereof, (for example, oleic acid), a bile salt, a phospholipid, an alkyl saccharide, a perfluorinated or polyethoxylated surfactant, or other pharmaceutically acceptable dispersant.

The compounds of the invention may also be administered by means of a dry powder inhaler. The inhaler may be a single or a multi dose inhaler, and may be a breath actuated dry powder inhaler.

One possibility is to mix the finely divided compound with a carrier substance, for example, a mono-, di- or polysaccharide, a sugar alcohol, or an other polyol. Suitable carriers are sugars, for example, lactose, glucose, raffinose, melezitose, lactitol, maltitol, trehalose, sucrose, mannitol; and starch. Alternatively the finely divided compound may be coated by another substance. The powder mixture may also be dispensed into hard gelatine capsules, each containing the desired dose of the active compound.

Another possibility is to process the finely divided powder into spheres which break up during the inhalation procedure. This spheronized powder may be filled into the drug reservoir of a multidose inhaler, for example, that known as the Turbuhaler^{®} in which a dosing unit meters the desired dose which is then inhaled by the patient. With this system the active compound, with or without a carrier substance, is delivered to the patient.

For oral administration the active compound may be admixed with an adjuvant or a carrier, for example, lactose, saccharose, sorbitol, mannitol; a starch, for example, potato starch, corn starch or amylopectin; a cellulose derivative; a binder, for example, gelatine or polyvinylpyrrolidone; and/or a lubricant, for example, magnesium stearate, calcium stearate, polyethylene glycol, a wax, paraffin, and the like, and then compressed into tablets. If coated tablets are required, the cores, prepared as described above, may be coated with a concentrated sugar solution which may contain, for example, gum arabic, gelatine, talcum, titanium dioxide, and the like. Alternatively, the tablet may be coated with a suitable polymer dissolved in a readily volatile organic solvent.

For the preparation of soft gelatine capsules, the compound may be admixed with, for example, a vegetable oil or polyethylene glycol. Hard gelatine capsules may contain granules of the compound using either the above mentioned excipients for tablets. Also liquid or semisolid formulations of the drug may be filled into hard gelatine capsules.

Liquid preparations for oral application may be in the form of syrups or suspensions, for example, solutions containing the compound, the balance being sugar and a mixture of ethanol, water, glycerol and propylene glycol. Optionally such liquid preparations may contain colouring agents, flavouring agents, saccharine and/or carboxymethylcellulose as a thickening agent or other excipients known to those skilled in art.

The compounds of the invention may also be administered in conjunction with other compounds used for the treatment of the above conditions.

The following Examples are intended to illustrate, but in no way limit the scope of the invention.

### General procedures

¹H NMR spectra were recorded on a Varian *Mercury-*VX 300 MHz instrument. The central peak of dimethylsulfoxide-*d₆* (δ_{H} 2.50 ppm) was used as internal reference. Column chromatography was carried out using silica gel (0.040-0.063 mm, Merck). Unless stated otherwise, starting materials were commercially available. All solvents and commercial reagents were of laboratory grade and were used as received. Unless otherwise stated, organic solutions were dried using anhydrous Na₂SO₄.
LC-MS Conditions : Instrument Agilent 1100; Column: Waters Symmetry 2.1 x 30 mm; C18 3.5µm; Mass APCI; Flow rate 0.7 ml/min; Wavelength 254 nm; Solvent A: water + 0.1 % TFA; solvent B: acetonitrile + 0.1 % TFA ; Gradient 15-95%/B 8 min, 95 % B 1 min. retention times (RT) are recorded in minutes.

The following abbreviations are used:
- HATU: O-(7-Azabenzotriazol-1-yl)-N,N,N',N'-tetramethyluronium hexafluorophosphate
- HOAT: 1-Hydroxy-7-azabenzotriazole
- DIEA: N,N-Diisopropylethylamine
- NMP: 1-N-Methyl-2-pyrrolidinone

### Example 1.1 N-(4-Chlorobenzyl)-3-oxo-4-[3-(trifluoromethyl)phenyl]-3,4-dihydroquinoxaline-2-carboxamide

### a) 3-Oxo-4-[3-(trifluoromethyl)phenyl]-3,4-dihydroquinoxaline-2-carboxylic acid

The title compound was prepared using the general method described by Hyrayama Takashi et al, J. Chem. Soc. Perkin Trans. 1, 1987, 75-84.
¹H NMR (DMSO-d₆): δ 14.03 (1H, s); 8.06-7.80 (5H, m); 7.56 (1H, t); 7.44 (1H, t); 6.62 (1H, d).

### b) N-(4-Chlorobenzyl)-3-oxo-4-[3-(trifluoromethyl)phenyl]-3,4-dihydroquinoxaline-2-carboxamide

A mixture of 3-oxo-4-[3-(trifluoromethyl)phenyl]-3,4-dihydroquinoxaline-2-carboxylic acid (94 mg, 0.28 mmol), HATU (122 mg, 0.32 mmol), HOAT (44 mg, 0.32 mmol) and DIEA (138 µl, 0.78 mmol) in NMP (3 ml) were added to 4-chlorobenzylamine (40 mg, 0.28 mmol) in NMP (2 ml). The reaction mixture was stirred for 18 h. The reaction was diluted with water (8 ml) and purified using preparative HPLC to give the title compound (63 mg, 49%).
¹H NMR (DMSO-d₆): δ 9.35 (1H, t); 8.04-7.90 (4H, m); 7.80 (1H, d); 7.57 (1H, t); 7.45 (1H, t); 7.41 (2H, s); 6.62 (1H, d); 4.51 (1H, d).

### Example 1.2 4-(3-Cyanophenyl)-N-[4-(methylsulfonyl)-benzyl]-3-oxo-3,4-dihydroquinoxaline-2-carboxamide

### a) 4-(3-Cyanophenyl)-3-oxo-3,4-dihydroquinoxaline-2-carboxylic acid

The title compound was prepared using the general method described by Hyrayama Takashi et al, J. Chem. Soc. Perkin Trans. 1, 1987, 75-84.
¹H NMR (DMSO-d₆): δ 14.04 (1H, s); 8.11 (2H, m); 7.95 (1H, d); 7.89 (2H, m); 7.56 (1H, t); 7.45 (1H, t); 6.65 (1H, d).

### b) 4-(3-Cyanophenyl)-N-[4-(methylsulfonyl)benzyl]-3-oxo-3,4-dihydroquinoxaline-2-carboxamide

The title compound was prepared by a method analogous to that described in Example 1.1.
¹H NMR (DMSO-d₆): δ 9.45 (1H, t); 8.11 (2H, dt); 7.99 (1H, dd); 7.94-7.82 (4H, m); 7.64 (2H, d); 7.57 (1H, dt); 7.46 (1H, dt); 6.68 (1H, d); 4.63 (2H, d); 3.20 (3H, s).

### Example 1.3 4-(3-Chlorophenyl)-N-[4-(methylsulfonyl)benzyl]-3-oxo-3,4-dihydroquinoxaline-2-carboxamide

### a) 4-(3-Chlorophenyl)-3-oxo-3,4-dihydroquinoxaline-2-carboxylic acid

The title compound was prepared using the general method described by Hyrayama Takashi et al, J. Chem. Soc. Perkin Trans. 1, 1987, 75-84.
¹H NMR (DMSO-d₆): δ 14.04 (1H, s); 7.94 (1H, d); 7.70 (3H, m); 7.56 (1H, t); 7.52-7.38 (2H, t); 6.66 (1H, d).

### b) 4-(3-Chlorophenyl)-N-[4-(methylsulfonyl)benzyl]-3-oxo-3,4-dihydroquinoxaline-2-carboxamide

The title compound was prepared by a method analogous to that described in Example 1.1.
¹H NMR (DMSO-d₆): δ 9.45 (1H, t); 7.97 (1H, d); 7.91 (2H, d); 7.77-7.51 (6H, m); 7.45 (2H, m); 6.68 (1H, d); 4.63 (2H, d); 3.20 (3H, s).

### Example 1.4 4-(3-Methylphenyl)-N-[4-(methylsulfonyl)benzyl]-3-oxo-3,4-dihydroquinoxaline-2-carboxamide

### a) 4-(3-Methylphenyl)-3-oxo-3.4-dihydroquinoxaline-2-carboxylic acid

The title compound was prepared using the general method described by Hyrayama Takashi et al, J. Chem. Soc. Perkin Trans. 1, 1987, 75-84.
¹H NMR (DMSO-d₆): δ 14.04 (1H, s); 7.93 (1H, dd); 7.55 (2H, m); 7.43 (2H, m); 7.25 (2H, d); 6.64 (1H, d); 2.41 (3H, s).

### b) 4-(3-Methylphenyl)-N-[4-(methylsulfonyl)benzyl]-3-oxo-3,4-dihydroquinoxaline-2-carboxamide

The title compound was prepared by a method analogous to that described in Example 1.1.
¹H NMR (DMSO-d₆): δ 9.46 (1H, t); 7.96 (1H, d); 7.91 (2H, d); 7.65 (2H, d); 7.56 (2H, t); 7.44 (2H, m); 7.22 (2H, d); 6.64 (1H, d); 4.62 (2H, d); 3.20 (3H, s); 2.41 (3H, s).

Using the appropriate amine or a salt thereof, the following compounds were prepared by a method analogous to that described in Example 1.1.

### Example 2.1 N-[4-(Methylsulfonyl)benzyl]-3-oxo-4-[3-(trifluoromethyl)phenyl]-3,4-dihydroquinoxaline-2-carboxamide

¹H NMR (DMSO-d₆): δ 9.44 (1H, t); 8.04-7.87 (6H, m); 7.81 (1H, d); 7.64 (2H, d); 7.57 (1H, dt); 7.46 (1H, dt); 6.64 (1H, d); 4.63 (1H, d); 3.20 (3H, s).

### Example 2.2 N-(Cyclohexylmethyl)-3-oxo-4-[3-(trifluoromethyl)phenyl]-3,4-dihydroquinoxaline-2-carboxamide

LC-MS RT: 5.76 min, m/z 430.2 [MH⁺].

### Example 2.3 N-[2-(3,4-Dimethoxyphenyl)ethyl]-3-oxo-4-[3-(trifluoromethyl)phenyl]-3,4-dihydroquinoxaline-2-carboxamide

LC-MS RT: 4.89 min, m/z 498.2 [MH⁺].

### Example 2.4 3-Oxo-N-(pyridin-3-ylmethyl)-4-[3-(trifluoromethyl)phenyl]-3,4-dihydroquinoxaline-2-carboxamide

LC-MS RT: 3.09 min, m/z 425.1 [MH⁺].

### Example 2.5 3-Oxo-N'-phenyl-4-[3-(trifluoromethyl)phenyl]-3,4-dihydroquinoxaline-2-carbohydrazide

LC-MS RT: 4.90 min, m/z 425.1 [MH⁺].

### Example 2.6 N-(3-Bromobenzyl)-3-oxo-4-[3-(trifluoromethyl)phenyl]-3,4-dihydroquinoxaline-2-carboxamide

LC-MS RT: 5.63 min, m/z 502.1 [MH⁺].

### Example 2.7 N-(4-Bromobenzyl)-3-oxo-4-[3-(trifluoromethyl)phenyl]-3,4-dihydroquinoxaline-2-carboxamide

LC-MS RT: 5.68 min, m/z 502.1 [MH⁺].

### Example 2.8 3-Oxo-N-[(2R)-2-phenylcyclopropyl]-4-[3-(trifluoromethyl)phenyl]-3,4-dihydroquinoxaline-2-carboxamide

LC-MS RT: 5.57 min, m/z 450.2 [MH⁺].

### Example 2.9 N-[2-(3-Chlorophenyl)ethyl]-3-oxo-4-[3-(trifluoromethyl)phenyl]-3,4-dihydroquinoxaline-2-carboxamide

LC-MS RT: 5.68 min, m/z 472.1 [MH⁺].

### Example 2.10 N-[(4-Cyanocyclohexyl)methyl]-3-oxo-4-[3-(trifluoromethyl)phenyl]-3,4-dihydroquinoxaline-2-carboxamide

LC-MS RT: 4.79 min, m/z 455.2 [MH⁺].

### Example 2.11 N-(1-Naphthylmethyl)-3-oxo-4-[3-(trifluoromethyl)phenyl]-3,4-dihydroquinoxaline-2-carboxamide

LC-MS RT: 5.71 min, m/z 474.2 [MH⁺].

### Example 2.12 N-(4-Methylbenzyl)-3-oxo-4-[3-(trifluoromethyl)phenyl]-3,4-dihydroquinoxaline-2-carboxamide

LC-MS RT: 5.47 min, m/z 438.2 [MH⁺].

### Example 2.13 N-(1,3-Benzodioxol-5-ylmethyl)-3-oxo-4-[3-(trifluoromethyl)phenyl]-3,4-dihydroquinoxaline-2-carboxamide

LC-MS RT: 5.05 min, m/z 468.1 [MH⁺].

### Example 2.14 N-(2,4-Dichlorobenzyl)-3-oxo-4-[3-(trifluoromethyl)phenyl]-3,4-dihydroquinoxaline-2-carboxamide

LC-MS RT: 5.98 min, m/z 492.1 [MH⁺].

### Example 2.15 N-(4-Methoxybenzyl)-3-oxo-4-[3-(trifluoromethyl)phenyl]-3,4-dihydroquinoxaline-2-carboxamide

LC-MS RT: 5.11 min, m/z 454.2 [MH⁺].

### Example 2.16 N-(3,4-Difluorobenzyl)-3-oxo-4-[3-(trifluoromethyl)phenyl]-3,4-dihydroquinoxaline-2-carboxamide

LC-MS RT: 5.38 min, m/z 460.1 [MH⁺].

### Example 2.17 N-(2-Chloro-4-fluorobenzyl)-3-oxo-4-[3-(trifluoromethyl)phenyl]-3,4-dihydroquinoxaline-2-carboxamide

APCI-MS m/z: 476.3 [MH⁺].

### Example 2.18 N-(3,4-Dichlorobenzyl)-3-oxo-4-[3-(trifluoromethyl)phenyl]-3,4-dihydroquinoxaline-2-carboxamide

APCI-MS m/z: 492.2 [MH⁺].

### Example 2.19 N-[2-(3-Methoxyphenyl)ethyl]-3-oxo-4-[3-(trifluoromethyl)phenyl]-3,4-dihydroquinoxaline-2-carboxamide

LC-MS RT: 6.80 min, m/z 468.4 [MH⁺].

### Example 2.20 N-[2-(4-Fluorophenyl)ethyl]-3-oxo-4-[3-(trifluoromethyl)phenyl]-3,4-dihydroquinoxaline-2-carboxamide

APCI-MS m/z: 456.3 [MH⁺].

### Example 2.21 N-[1-(4-Chlorophenyl)ethyl]-3-oxo-4-[3-(trifluoromethyl)phenyl]-3,4-dihydroquinoxaline-2-carboxamide

APCI-MS m/z: 472.3 [MH⁺].

### Example 2.22 3-Oxo-N-[3-(2-oxopyrrolidin-1-yl)propyl]-4-[3-(trifluoromethyl)phenyl]-3,4-dihydroquinoxaline-2-carboxamide

APCI-MS m/z: 459.3 [MH⁺].

### Example 2.23 3-Oxo-N-(pyridin-4-ylmethyl)-4-[3-(trifluoromethyl)phenyl]-3,4-dihydroquinoxaline-2-carboxamide

LC-MS RT: 3.12 min, m/z 425.1 [MH⁺].

### Example 2.24 N-(2,3-Dihydro-1-benzofuran-5-ylmethyl)-3-oxo-4-[3-(trifluoromethyl)phenyl]-3,4-dihydroquinoxaline-2-carboxamide

LC-MS RT: 5.10 min, m/z 466.2 [MH⁺].

### Example 2.25 Methyl 4-{[({3-oxo-4-[3-(trifluoromethyl)phenyl]-3,4-dihydroquinoxalin-2-yl}carbonyl)amino]methyl}benzoate

LC-MS RT: 5.08 min, m/z 482.2 [MH⁺].

### Example 2.26 3-Oxo-N-(4-phenoxybenzyl)-4-[3-(trifluoromethyl)phenyl]-3,4-dihydroquinoxaline-2-carboxamide

APCI-MS m/z: 516.3 [MH⁺].

### Example 2.27 N-{2-[4-(Aminosulfonyl)phenyl]ethyl}-3-oxo-4-[3-(trifluoromethyl)phenyl]-3,4-dihydroquinoxaline-2-carboxamide

APCI-MS m/z: 517.3 [MH⁺].

### Example 2.28 3-Oxo-N-[4-(1H-pyrazol-1-yl)benzyl]-4-[3-(trifluoromethyl)phenyl]-3,4-dihydroquinoxaline-2-carboxamide

LC-MS RT: 4.99 min, m/z 490.2 [MH⁺].

### Example 2.29 3-Oxo-N-phenoxy-4-[3-(trifluoromethyl)phenyl]-3,4-dihydroquinoxaline-2-carboxamide

LC-MS RT: 5.11 min, m/z 426.1 [MH⁺].

### Example 2.30 N-{[3-(4-Methoxyphenyl)isoxazol-5-yl]methyl}-3-oxo-4-[3-(trifluoromethyl)phenyl]-3,4-dihydroquinoxaline-2-carboxamide

LC-MS RT: 5.40 min, m/z 521.2 [MH⁺].

### Example 2.31 N-(3-Azepan-1-ylpropyl)-3-oxo-4-[3-(trifluoromethyl)phenyl]-3,4-dihydroquinoxaline-2-carboxamide

LC-MS RT: 3.50 min, m/z 473.3 [MH⁺].

### Example 2.32 N-[4-(Acetylamino)benzyl]-3-oxo-4-[3-(trifluoromethyl)phenyl]-3,4-dihydroquinoxaline-2-carboxamide

LC-MS RT: 4.20 min, m/z 481.2 [MH⁺].

### Example 2.33 N-(4-Cyanobenzyl)-3-oxo-4-[3-(trifluoromethyl)phenyl]-3,4-dihydroquinoxaline-2-carboxamide

LC-MS RT: 4.94 min, m/z 449.1 [MH⁺].

### Example 2.34 N'-[4-(Methylsulfonyl)phenyl]-3-oxo-4-[3-(trifluoromethyl)phenyl]-3,4-dihydroquinoxaline-2-carbohydrazide

LC-MS RT: 4.31 min, m/z 503.1 [MH⁺].

### Example 2.35 N-[4-(Methylsulfonyl)phenoxy]-3-oxo-4-[3-(trifluoromethyl)phenyl]-3,4-dihydroquinoxaline-2-carboxamide

LC-MS RT: 4.53 min, m/z 504.1 [MH⁺].

### Screen

### Human Neutrophil Elastase Quenched-FRET Assay

The assay uses Human Neutrophil Elastase (HNE) purified from serum (Calbiochem art. 324681; Ref. Baugh, R.J. et al., 1976, Biochemistry. 15, 836-841). HNE was stored in 50 mM NaOAc, 200 mM NaCl, pH 5.5 with added 30% glycerol at -20°C. The protease substrate used was Elastase Substrate V Fluorogenic, MeOSuc-AAPV-AMC (Calbiochem art. 324740; Ref. Castillo, M.J. et al., 1979, Anal. Biochem. 99, 53-64). The substrate was stored in DMSO at -20°C. The assay additions were as follows: Test compounds and controls were added to black 96-well flat-bottom plates (Greiner 655076), 1 µL in 100% DMSO, followed by 30 µL HNE in assay buffer with 0.01% TritonX-100. The assay buffer constitution was: 100 mM Tris (pH 7.5) and 500 mM NaCl. The enzyme and the compounds were incubated at room temperature for 15 minutes. Then 30 µl substrate in assay buffer was added. The assay was stopped after 30 minutes incubation at room temperature by adding 60 µl stop solution (140 mM acetic acid, 200 mM sodium monochloroacetate, 60 mM sodium acetate, pH 4.3). Fluorescence was measured on a Wallac 1420 Victor 2 instrument at settings: Excitation 380 nm, Emission 460 nm. IC₅₀ values were determined using Xlfit curve fitting using model 205.

When tested in the above screen, the compounds of the Examples gave IC₅₀ values for inhibition of human neutrophil elastase activity of less than 30 µM, indicating that the compounds of the invention are expected to possess useful therapeutic properties. Specimen results are shown in the following Table:

| Compound | Inhibition of Human Neutrophil Elastase IC₅₀(nM) |
|---|---|
| Example 2.12 | 226 |
| Example 2.10 | 248 |
| Example 2.20 | 573 |

## Claims

1. A compound of formula (I) 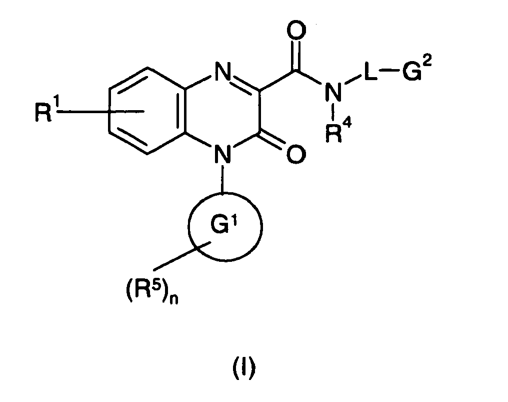 wherein
**R¹** represents H, halogen, CN, C1 to 6 alkyl, C1 to 6 alkoxy, CO₂R⁷ or CONR⁸R⁹;
**G¹** represents phenyl or a five- or six-membered heteroaromatic ring containing 1 to 3 heteroatoms independently selected from O, S and N;
**R⁵** represents H, halogen, C1 to 6 alkyl, CN, C1 to 6 alkoxy, NO₂, NR¹⁴R¹⁵, C1 to 3 alkyl substituted by one or more F atoms or C1 to 3 alkoxy substituted by one or more F atoms;
**R¹⁴** and **R¹⁵** independently represent H or C1 to 3 alkyl; said alkyl being optionally further substituted by one or more F atoms;
**n** represents an integer 1, 2 or 3 and when n represents 2 or 3, each R⁵ group is selected independently;
**R⁴** represents H or C1 to 6 alkyl; said alkyl being optionally further substituted by OH or C1 to 6 alkoxy;
or **R⁴** and **L** are joined together such that the group **-NR⁴L** represents a 5 to 7 membered azacyclic ring optionally incorporating one further heteroatom selected from O, S and NR¹⁶;
**L** represents a bond, O, NR²⁹ or C1 to 6 alkyl; said alkyl being optionally incorporating a heteroatom selected from O, S and NR¹⁶; and said alkyl being optionally further substituted by OH or OMe;
**G²** represents a monocyclic ring system selected from:
i) phenyl or phenoxy,
ii) a 5 or 6 membered heteroaromatic ring containing one to three heteroatoms independently selected from O, S and N,
iii) a C3 to 6 saturated or partially unsaturated cycloalkyl, or
iv) a C4 to 7 saturated or partially unsaturated heterocyclic ring containing one or two heteroatoms independently selected from O, S(O)ₚ and NR¹⁷ and optionally further incorporating a carbonyl group; or
**G²** represents a bicyclic ring system in which each of the two rings is independently selected from:
i) phenyl,
ii) a 5 or 6 membered heteroaromatic ring containing one to three heteroatoms independently selected from O, S and N,
iii) a C3 to 6 saturated or partially unsaturated cycloalkyl, or
iv) a C4 to 7 saturated or partially unsaturated heterocyclic ring containing one or two heteroatoms independently selected from O, S(O)ₚ and NR¹⁷ and optionally further incorporating a carbonyl group;
and the two rings are either fused together, or are bonded directly together or are separated by a linker group selected from O, S(O)_{q} or CH₂,
said monocyclic or bicyclic ring system being optionally further substituted by one to three substituents independently selected from CN, OH, C1 to 6 alkyl, C1 to 6 alkoxy, halogen, NR¹⁸R¹⁹, NO₂, OSO₂R³⁸, CO₂R²⁰, C(=NH)NH₂, C(O)NR²¹R²², C(S)NR²³R²⁴, SC(=NH)NH₂, NR³¹C(=NH)NH₂, S(O)ₛR²⁵, SO₂NR²⁶R²⁷, C1 to 3 alkoxy substituted by one or more F atoms and C1 to 3 alkyl substituted by SO₂R³⁹ or by one or more F atoms; or
when L does not represent a bond, **G²** may also represent H;
**p, q, s** and **t** independently represent an integer 0, 1 or 2;
**R⁸** and **R⁹** independently represent H or C1 to 6 alkyl; or the group **NR⁸R⁹** together represents a 5 to 7 membered azacyclic ring optionally incorporating one further heteroatom selected from O, S and NR²⁸;
**R¹⁸** and **R¹⁹** independently represent H, C1 to 6 alkyl, formyl, C2 to 6 alkanoyl, S(O)ₜR³² or SO₂NR³³R³⁴; said alkyl group being optionally further substituted by halogen, CN, C1 to 4 alkoxy or CONR⁴¹R⁴²;
**R²⁵** represents H, C1 to 6 alkyl or C3 to 6 cycloalkyl; said alkyl group being optionally further substituted by one or more substituents selected independently from OH, CN, CONR³⁵R³⁶, CO₂R³⁷, OCOR⁴⁰, C3 to 6 cycloalkyl, a C4 to 7 saturated heterocyclic ring containing one or two heteroatoms independently selected from O, S(O)ₚ and NR⁴³ and phenyl or a 5 or 6 membered heteroaromatic ring containing one to three heteroatoms independently selected from O, S and N; said aromatic ring being optionally further substituted by one or more substituents selected independently from halogen, CN, C1 to 4 alkyl, C1 to 4 alkoxy, OH, CONR⁴⁴R⁴⁵, CO2R⁴⁶, S(O)ₛR²⁵ or NHCOCH₃;
**R³²** represents H, C1 to 6 alkyl or C3 to 6 cycloalkyl;
**R⁷, R¹⁶, R¹⁷, R²⁰, R²¹, R²², R²³, R²⁴, R²⁶, R²⁷, R²⁸, R²⁹, R³¹, R³³, R³⁴, R³⁵, R³⁶, R³⁷, R³⁸, R³⁹, R⁴⁰, R⁴¹, R⁴², R⁴³, R⁴⁴, R⁴⁵ and R⁴⁶** independently represent H or C1 to 6 alkyl;
and pharmaceutically acceptable salts thereof.

2. A compound of formula (I), according to Claim 1, wherein G¹ represents phenyl.

3. A compound of formula (I), according to Claim 1 or Claim 2, wherein R⁴ represents H.

4. A compound of formula (I), according to any one of Claims 1 to 3, wherein R⁵ represents Cl, CH₃, CN or CF₃.

5. A compound of formula (I), according to any one of Claims 1 to 4, or a pharmaceutically acceptable salt thereof, for use as a medicament.

6. A pharmaceutical formulation comprising a compound of formula (I), as defined in any one of Claims 1 to 4, or a pharmaceutically acceptable salt thereof, optionally in admixture with a pharmaceutically acceptable diluent or carrier.

7. The use of a compound of formula (I) as defined in any one of Claims 1 to 4, or a pharmaceutically acceptable salt thereof, in the manufacture of a medicament for the treatment or prophylaxis of inflammatory diseases or conditions.

8. The use of a compound of formula (I) as defined in any one of Claims 1 to 4, or a pharmaceutically acceptable salt thereof, in the manufacture of a medicament for the treatment or prophylaxis of adult respiratory distress syndrome (ARDS), cystic fibrosis, pulmonary emphysema, chronic obstructive pulmonary disease (COPD), pulmonary hypertension, asthma, rhinitis, ischemia-reperfusion injury, rheumatoid arthritis, osteoarthritis, cancer, atherosclerosis and gastric mucosal injury.

9. A process for the preparation of a compound of formula (I), as defined in any one of Claims 1 to 4, and optical isomers, racemates and tautomers thereof and pharmaceutically acceptable salts thereof, which comprises reacting a compound of formula (II) 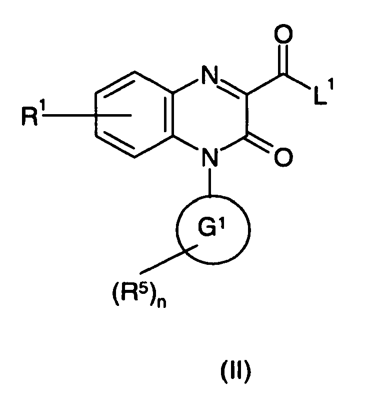 wherein R¹, R⁵, G¹ and n are as defined in Claim 1 and L¹ represents a leaving group, with an amine of formula (III) or a salt thereof 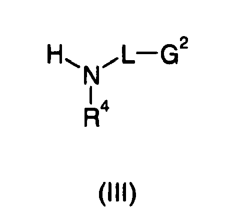 wherein R⁴, G² and L are as defined in Claim 1,
and where desired or necessary converting the resultant compound of formula (I), or another salt thereof, into a pharmaceutically acceptable salt thereof; or converting one compound of formula (I) into another compound of formula (I); and where desired converting the resultant compound of formula (I) into an optical isomer thereof.

## Patentansprüche

1. Verbindungen der Formel (I) 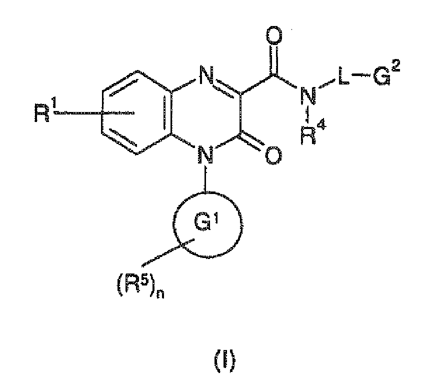 wobei
R¹ für H, Halogen, CN, C₁₋₆-Alkyl, C₁₋₆-Alkoxy, CO₂R⁷ oder CONR⁸R⁹ steht;
G¹ für Phenyl oder einen fünf- oder sechsgliedrigen heteroaromatischen Ring mit 1 bis 3 Heteroatomen unabhängig voneinander ausgewählt aus 0, S und N steht;
R⁵ für H, Halogen, C₁₋₆-Alkyl, CN, C₁₋₆-Alkoxy, NO₂, NR¹⁴R¹⁵, durch ein oder mehrere F-Atome substituiertes C₁₋₃-Alkyl oder durch ein oder mehrere F-Atome substituiertes C₁₋₃-Alkoxy steht;
R¹⁴ und R¹⁵ unabhängig voneinander für H oder C₁₋₃-Alkyl stehen; wobei das Alkyl gegebenenfalls weiter durch ein oder mehrere F-Atome substituiert ist;
n für eine ganze Zahl 1, 2 oder 3 steht und, wenn n für 2 oder 3 steht, die R⁵-Gruppen jeweils unabhängig voneinander ausgewählt sind;
R⁴ für H oder C₁₋₆-Alkyl steht; wobei das Alkyl gegebenenfalls weiter durch OH oder C₁₋₆-Alkoxy substituiert ist;
oder R⁴ und L so miteinander verbunden sind, daß die Gruppe -NR⁴L für einen 5- bis 7gliedrigen azacyclischen Ring steht, der gegebenenfalls ein weiteres Heteroatom ausgewählt aus 0, S und NR¹⁶ einschließt;
L für eine Bindung, 0, NR²⁹ oder C₁₋₆-Alkyl steht; wobei das Alkyl gegebenenfalls ein Heteroatom ausgewählt aus O, S und NR¹⁶ einschließt; und wobei das Alkyl gegebenenfalls weiter durch OH oder OMe substituiert ist;
G² für ein monocyclisches Ringsystem steht, ausgewählt aus:
i) Phenyl oder Phenoxy,
ii) einem 5- oder 6gliedrigen heteroaromatischen Ring mit einem bis drei Heteroatomen unabhängig voneinander ausgewählt aus 0, S und N,
iii) einem gesättigten oder teilweise ungesättigten C₃₋₆-Cycloalkyl, oder
iv) einem gesättigten oder teilweise ungesättigten heterocyclischen C₄₋₇-Ring mit einem oder zwei Heteroatomen unabhängig voneinander ausgewählt aus O, S(O)ₚ und NR¹⁷, der gegebenenfalls weiter eine Karbonylgruppe einschließt; oder
G² für ein bicyclisches Ringsystem steht, in dem die beiden Ringe jeweils unabhängig voneinander ausgewählt sind aus:
i) Phenyl,
ii) einem 5- oder 6gliedrigen heteroaromatischen Ring mit einem bis drei Heteroatomen unabhängig voneinander ausgewählt aus O, S und N,
iii) einem gesättigten oder teilweise ungesättigten C₃₋₆-Cycloalkyl, oder
iv) einem gesättigten oder teilweise ungesättigten heterocyclischen C₄₋₇-Ring mit einem oder zwei Heteroatomen unabhängig voneinander ausgewählt aus O, S(O)ₚ und NR¹⁷, der gegebenenfalls weiter eine Carbonylgruppe einschließt;
und die beiden Ringe entweder miteinander kondensiert sind oder direkt aneinander gebunden sind oder durch eine aus O, S(O)_{q} oder CH₂ ausgewählte Brücke getrennt sind,
wobei das monocyclische oder bicyclische Ringsystem gegebenenfalls weiter substituiert ist durch einen bis drei Substituenten unabhängig voneinander ausgewählt aus CN, OH, C₁₋₆-Alkyl, C₁₋₆-Alkoxy, Halogen, NR¹⁸R¹⁹, NO₂, OSO₂R³⁸, CO₂R²⁰, C(=NH)NH₂, C (0) NR²¹R²², C (S) NR²³R²⁴, SC (=NH) NH₂, NR³¹C (=NH) NH₂, S(O)ₛR²⁵, SO₂NR²⁶R²⁷, durch ein oder mehrere F-Atome substituiertes C₁₋₃-Alkoxy und durch SO₂R³⁹ oder durch ein oder mehrere F-Atome substituiertes C₁₋₃-Alkyl; oder
wenn L nicht für eine Bindung steht, G² auch für H stehen kann;
p, q, s und t unabhängig voneinander für eine ganze Zahl 0, 1 oder 2 stehen;
R⁸ und R⁹ unabhängig voneinander für H oder C₁₋₆-Alkyl stehen; oder die Gruppe NR⁸R⁹ zusammen für einen 5- bis 7gliedrigen azacyclischen Ring steht, der gegebenenfalls ein weiteres Heteroatom ausgewählt aus O, S und NR²⁸ einschließt;
R¹⁸ und R¹⁹ unabhängig voneinander für H, C₁₋₆-Alkyl, Formyl, C₂₋₆-Alkanoyl, S(O)ₜR³² oder SO₂NR³³R³⁴ stehen; wobei die Alkylgruppe gegebenenfalls weiter durch Halogen, CN, C₁₋₄-Alkoxy oder CONR⁴¹R⁴² substituiert ist;
R²⁵ für H, C₁₋₆-Alkyl oder C₃₋₆-Cycloalkyl steht; wobei die Alkylgruppe gegebenenfalls weiter durch einen oder mehrere Substituenten unabhängig voneinander ausgewählt aus OH, CN, CONR³⁵R³⁶, CO²R³⁷, OCOR⁴⁰, C₃₋₆-Cycloalkyl, einen gesättigten heterocyclischen C₄₋₇-Ring mit einem oder zwei Heteroatomen unabhängig voneinander ausgewählt aus O, S(O)ₚ und NR⁴³ und Phenyl oder einen 5- oder 6-gliedrigen heteroaromatischen Ring mit einem bis drei Heteroatomen unabhängig voneinander ausgewählt aus O, S und N substituiert ist; wobei der aromatische Ring gegebenenfalls weiter durch einen oder mehrere Substituenten unabhängig voneinander ausgewählt aus Halogen, CN, C₁₋₄-Alkyl, C₁₋₄-Alkoxy, OH, CONR⁴⁴R⁴⁵, CO2R⁴⁶, S(O)ₛR²⁵ oder NHCOCH₃ substituiert ist;
R³² für H, C₁₋₆-Alkyl oder C₃₋₆-Cycloalkyl steht;
R⁷, R¹⁶, R¹⁷, R²⁰, R²¹, R²², R²³, R²⁴, R²⁶, R²⁷, R²⁸, R²⁹, R³¹, R³³, R³⁴, R³⁵, R³⁶, R³⁷, R³⁸, R³⁹, R⁴⁰, R⁴¹, R⁴², R⁴³, R⁴⁴, R⁴⁵ und R⁴⁶ unabhängig voneinander für H oder C₁₋₆-Alkyl stehen;
und deren pharmazeutisch annehmbare Salze.

2. Verbindungen der Formel (I) nach Anspruch 1, wobei G¹ für Phenyl steht.

3. Verbindungen der Formel (I) nach Anspruch 1 oder 2, wobei R⁴ für H steht.

4. Verbindungen der Formel (I) nach einem der Ansprüche 1 bis 3, wobei R⁵ für Cl, CH₃, CN oder CF₃ steht.

5. Verbindungen der Formel (I) nach einem der Ansprüche 1 bis 4 und deren pharmazeutisch annehmbare Salze zur Verwendung als Medikament.

6. Pharmazeutische Formulierung, enthaltend eine wie in einem der Ansprüche 1 bis 4 definierte Verbindung der Formel (I) oder ein pharmazeutisch annehmbares Salz davon, gegebenenfalls in einer Mischung mit einem pharmazeutisch annehmbaren Verdünnungsmittel oder Träger.

7. Verwendung einer wie in einem der Ansprüche 1 bis 4 definierten Verbindung der Formel (I) oder eines pharmazeutisch annehmbaren Salzes davon bei der Herstellung eines Medikaments zur Behandlung oder Prophylaxe von entzündlichen Krankheiten oder Leiden.

8. Verwendung einer wie in einem der Ansprüche 1 bis 4 definierten Verbindung der Formel (I) oder eines pharmazeutisch annehmbaren Salzes davon bei der Herstellung eines Medikaments zur Behandlung oder Prophylaxe von akutem Atemnotsyndrom (Adult Respiratory Distress Syndrome, ARDS), cystischer Fibrose, Lungenemphysem, chronisch obstruktiver Atemwegserkrankung (Chronic Obstructive Pulmonary Disease, COPD), pulmonaler Hypertonie, Asthma, Rhinitis, ischämischer Reperfusionsverletzung, rheumatoider Arthritis, Osteoarthritis, Krebs, Atherosklerose und Magenschleimhautverletzung.

9. Verfahren zur Herstellung von wie in einem der Ansprüche 1 bis 4 definierten Verbindungen der Formel (I) und optischen Isomeren, Racematen und Tautomeren davon und pharmazeutisch annehmbaren Salzen davon, bei dem man eine Verbindung der Formel (II) 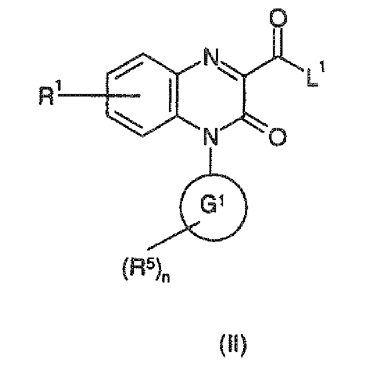 in welcher R¹, R⁵, G¹ und n wie in Anspruch 1 definiert sind und L¹ für eine Abgangsgruppe steht, mit einem Amin der Formel (III) oder einem Salz davon 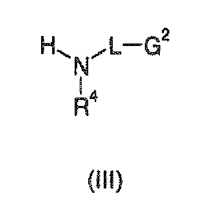 in welcher R⁴, G² und L wie in Anspruch 1 definiert sind, umsetzt,
und, falls gewünscht oder erforderlich, die auf diese Weise erhaltene Verbindung der Formel (I) oder ein anderes Salz davon in ein pharmazeutisch annehmbares Salz davon umwandelt; oder eine Verbindung der Formel (I) in eine andere Verbindung der Formel (I) umwandelt; und, falls gewünscht, die auf diese Weise erhaltene Verbindung der Formel (I) in ein optisches Isomer davon umwandelt.

## Revendications

1. Composé de formule (I) 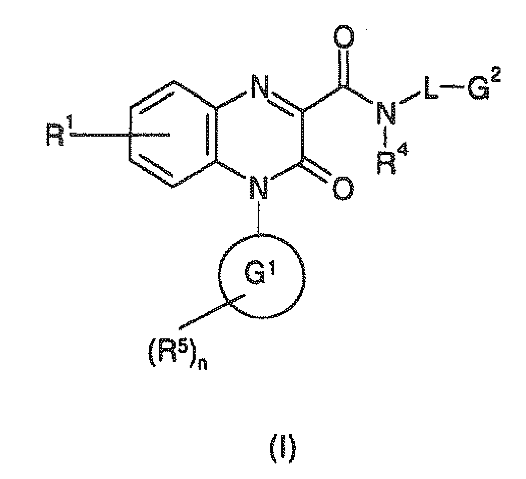 dans laquelle
**R¹** représente H, halogène, CN, alkyle en C1 à 6, alcoxy en C1 à 6, CO₂R⁷ ou CONR⁸R⁹ ;
**G¹** représente phényle ou un cycle hétéroaromatique à 5 ou 6 chaînons contenant de 1 à 3 hétéroatomes choisis indépendamment parmi 0, S et N ;
**R⁵** représente H, halogène, alkyle en C1 à 6, CN, alcoxy en C1 à 6, NO₂, NR¹⁴R¹⁵, alkyle en C1 à 3 substitué par un ou plusieurs atomes de F ou alcoxy en C1 à 3 substitué par un ou plusieurs atomes de F ;
**R¹⁴** et **R¹⁵** représentent indépendamment H ou alkyle en C1 à 3 ; ledit alkyle étant encore éventuellement substitué par un ou plusieurs atomes de F ;
**n** représente un entier 1, 2 ou 3 et lorsque n représente 2 ou 3, chaque groupement R⁵ est choisi indépendamment ;
**R⁴** représente H ou alkyle en C1 à 6 ; ledit alkyle étant encore éventuellement substitué par OH ou alcoxy en C1 à 6 ;
ou **R⁴** et **L** sont liés ensemble de sorte que le groupement **-NR⁴L** représente un cycle azacyclique à 5 à 7 chaînons comportant éventuellement un autre hétéroatome choisi parmi 0, S et NR¹⁶ ;
**L** représente une liaison, O, NR²⁹ ou alkyle en C1 à 6 ; ledit alkyle comportant éventuellement un hétéroatome choisi parmi O, S et NR¹⁶ ; ledit alkyle étant encore éventuellement substitué par OH ou OMe ;
**G²** représente un système de cycle monocyclique choisi parmi :
i) phényle ou phénoxy,
ii) un cycle hétéroaromatique à 5 ou 6 chaînons contenant de 1 à 3 hétéroatomes choisis indépendamment parmi O, S et N,
iii) un cycloalkyle en C3 à 6 saturé ou partiellement insaturé, ou
iv) un cycle hétérocyclique en C4 à 7 saturé ou partiellement insaturé contenant un ou deux hétéroatomes choisis indépendamment parmi O, S(O)ₚ et NR¹⁷ et comportant encore éventuellement un groupement carbonyle ; ou
**G²** représente un système de cycle bicyclique dans lequel chacun des deux cycles est choisi indépendamment parmi :
i) phényle,
ii) un cycle hétéroaromatique à 5 ou 6 chaînons contenant de 1 à 3 hétéroatomes choisis indépendamment parmi O, S et N,
iii) un cycloalkyle en C3 à 6 saturé ou partiellement insaturé, ou
iv) un cycle hétérocyclique en C4 à 7 saturé ou partiellement insaturé contenant un ou deux hétéroatomes choisis indépendamment parmi O, S(O)ₚ et NR¹⁷ et comportant encore éventuellement un groupement carbonyle ;
et les deux cycles sont soit fusionnés ensemble, soit liés directement ensemble, soit séparés par un groupement lieur choisi parmi O, S(O)_{q} ou CH₂,
ledit système de cycle monocyclique ou bicyclique étant éventuellement encore substitué par 1 à 3 substituants choisis indépendamment parmi CN, OH, alkyle en C1 à 6, alcoxy en C1 à 6, halogène, NR¹⁸R¹⁹, NO₂, OSO₂R³⁸, CO₂R²⁰, C(=NH)NH₂, C(O)NR²¹R²², C(S)NR²³R²⁴, SC(=NH)NH₂, NR³¹C (=NH) NH₂, S (O)ₛR²⁵, SO₂NR²⁶R²⁷, alcoxy en C1 à 3 substitué par un ou plusieurs atomes de F et alkyle en C1 à 3 substitué par SO₂R³⁹ ou par un ou plusieurs atomes de F ; ou
lorsque L ne représente pas une liaison, **G²** peut également représenter H ;
**p, q, s** et **t** représentent indépendamment un entier 0, 1 ou 2 ;
**R⁸** et **R⁹** représentent indépendamment H ou alkyle en C1 à 6 ; ou le groupement **NR⁸R⁹** représente ensemble un cycle azacyclique à 5 à 7 chaînons comportant éventuellement un autre hétéroatome choisi parmi 0, S et NR²⁸ ;
**R¹⁸** et **R¹⁹** représentent indépendamment H, alkyle en C1 à 6, formyle, alcanoyle en C2 à 6, S(O)ₜR³² ou SO₂NR³³R³⁴; ledit groupement alkyle étant éventuellement encore substitué par halogène, CN, alcoxy en C1 à 4 ou CONR⁴¹R⁴² ;
**R²⁵** représente H, alkyle en C1 à 6 ou cycloalkyle en C3 à 6 ; ledit groupement alkyle étant éventuellement encore substitué par un ou plusieurs substituants choisis indépendamment parmi OH, CN, CONR³⁵R³⁶, CO₂R³⁷, OCOR⁴⁰, cycloalkyle en C3 à 6, un cycle hétérocyclique en C4 à 7 saturé contenant un ou deux hétéroatomes choisis indépendamment parmi O, S(O)ₚ et NR⁴³ et phényle ou un cycle hétéroaromatique à 5 ou 6 chaînons contenant de 1 à 3 hétéroatomes choisis indépendamment parmi 0, S et N ; ledit cycle aromatique étant éventuellement encore substitué par un ou plusieurs substituants choisis indépendamment parmi halogène, CN, alkyle en C1 à 4, alcoxy en C1 à 4, OH, CONR⁴⁴R⁴⁵, CO₂R⁴⁶, S(O)ₛR²⁵ ou NHCOCH₃ ;
**R³²** représente H, alkyle en C1 à 6 ou cycloalkyle en C3 à 6 ;
**R**⁷, **R**¹⁶, **R**¹⁷, **R**²⁰, **R**²¹, **R**²², **R**²³, **R**²⁴, **R**²⁶, **R**²⁷, **R**²⁸, **R**²⁹, **R**³¹**, R**³³**, R**³⁴**, R**³⁵**, R**³⁶**, R**³⁷**, R**³⁸**, R**³⁹**, R**⁴⁰**, R**⁴¹**, R**⁴²**, R**⁴³, **R**⁴⁴, **R**⁴⁵, et **R**⁴⁶ représentent indépendamment H ou alkyle en C1 à 6 ;
et des sels pharmaceutiquement acceptables de celui-ci .

2. Composé de formule (I), selon la revendication 1, **caractérisé en ce que** G¹ représente phényle.

3. Composé de formule (I), selon la revendication 1 ou la revendication 2, **caractérisé en ce que** R⁴ représente H.

4. Composé de formule (I), selon l'une quelconque des revendications 1 à 3, **caractérisé en ce que** R⁵ représente Cl, CH₃, CN ou CF₃.

5. Composé de formule (I), selon l'une quelconque des revendications 1 à 4, ou un sel pharmaceutiquement acceptable de celui-ci, destiné à être utilisé comme médicament.

6. Formulation pharmaceutique comprenant un composé de formule (I), tel que défini dans l'une quelconque des revendications 1 à 4, ou un sel pharmaceutiquement acceptable de celui-ci, éventuellement en mélange avec un diluant ou un support pharmaceutiquement acceptable.

7. Utilisation d'un composé de formule (I), tel que défini dans l'une quelconque des revendications 1 à 4, ou d'un sel pharmaceutiquement acceptable de celui-ci, dans la fabrication d'un médicament destiné au traitement ou à la prophylaxie de maladies ou d'affections inflammatoires.

8. Utilisation d'un composé de formule (I), tel que défini dans l'une quelconque des revendications 1 à 4, ou d'un sel pharmaceutiquement acceptable de celui-ci, dans la fabrication d'un médicament destiné au traitement ou à la prophylaxie du syndrome de détresse respiratoire de l'adulte (SDRA), de la mucoviscidose, de l'emphysème pulmonaire, de la bronchopneumopathie chronique obstructive (BPCO), de l'hypertension pulmonaire, de l'asthme, de la rhinite, de lésions d'ischémie-reperfusion, de la polyarthrite rhumatoïde, de l'ostéoarthrite, du cancer, de l'athérosclérose et de lésions de la muqueuse gastrique.

9. Procédé de préparation d'un composé de formule (I), tel que défini dans l'une quelconque des revendications 1 à 4, et des isomères optiques, des racémates et des tautomères de celui-ci et des sels pharmaceutiquement acceptables de celui-ci, qui comprend la réaction d'un composé de formule (II) 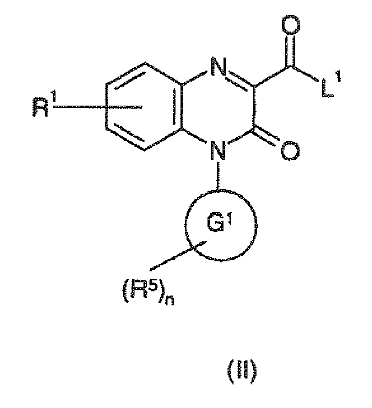 dans laquelle R¹, R⁵, G¹ et n sont tels que définis dans la revendication 1 et L¹ représente un groupement partant,
avec une amine de formule (III) ou un sel de celle-ci 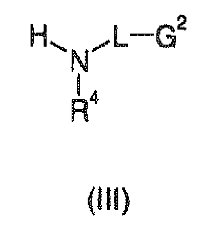 dans laquelle R⁴, G² et L sont tels que définis dans la revendication 1,
et, si on le souhaite ou si nécessaire, la transformation du composé résultant de formule (I), ou d'un autre sel de celui-ci, en un sel pharmaceutiquement acceptable de celui-ci ; ou la transformation d'un composé de formule (I) en un autre composé de formule (I) ; et si on le souhaite la transformation du composé résultant de formule (I) en un isomère optique de celui-ci.
